# EUROPEAN PATENT APPLICATION

(11) **EP 2 423 200 A1**
(43) Date of publication of application: **29.02.2012**
(21) Application number: 10163962.3
(22) Date of filing: 26.05.2010
(51) Int. Cl.: C07D 235/28

(54) **Polymorphic forms of 5-ethoxy-2-[2-(morpholino)-ethylthio]benzimidazole dihydrochloride and processes for their preparation**

(71) Applicant: AFOFARM, CJSC, Russia 141700 (RU)
(72) Inventor: Actins, Andris, Riga, LV-1057 (LV); Auzins, Raimons, Riga, LV-1057 (LV); Petkune, Sanita, Riga, LV-1057 (LV)
(74) Representative: Lavrinovics, Edvards

(57) **Abstract**

Polymorphic forms of 5-ethoxy-2-[2-(morpholino)-ethylthio]benzimidazole dihydrochloride commonly known as Afobazole and processes for their preparation.

## Description

### Technical Field

The invention provides a novel, polymorphic forms of 5-ethoxy-2-[2-(morpholino)-ethylthio]benzimidazole dihydrochloride and a process for their preparation.

### Background Art

5-Ethoxy-2-[2-(morpholino)-ethylthio]benzimidazole dihydrochloride commonly known as Afobazole.

Afobazole was first disclosed in EP 0788795 B (NAUCHNO-ISSLEDOVATELSKY INSTITUT FARMAKOLOGII AKADEMII MEDITSINSKIKH NAUK) 13.11.2002. Afobazole is an anxiolytic drug launched in Russia in the early 2000s.

The physicochemical properties of Afobazole were described in MILIKINA, S.E., et al. Analysis and standardization of the new anxiolytic drug Afobazole. Pharmaceutical Chemistry Journal, 2006, vol.40, no.7, p. 405-406. It was written that Afobazole appears as crystalline powder of white color, sometimes with a creamy tint, readily soluble in water (1g per 5-8 ml), as well hygroscopicity of substance was analyzed and the water content in saturated samples was about 12%. Stability tests of Afobazole were performed at natural conditions and using the accelerated test at 60°C what corresponding to two years under the natural conditions, and was concluded that the maximum storage time of Afobazole was 2 years.

Polymorphism is important in the development of pharmaceutical ingredients. Polymorphism in drugs can also have direct medical implications. Medicine is often administered orally as a crystalline solid and dissolution rates depend on the exact crystal form of a polymorph.

Surprisingly it has been found that Afobazole can exist in at least four novel crystalline forms and which have very advantageous properties.

Polymorphic forms I, II, III and IV of Afobazole is less hygroscopic than Afobazole.

The lower hygroscopicity is a further advantage for processing and storing Polymorphic forms I, II, III and IV of Afobazole.

### Brief description of drawings

Fig. 1 shows the X-ray diffraction pattern of 5-ethoxy-2-[2-(morpholino)-ethylthio]benzimidazole dihydrochloride crystal Form I
Fig. 2 shows the FT-IR spectrum of 5-ethoxy-2-[2-(morpholino)-ethylthio]benzimidazole dihydrogenchloride crystal Form I
Fig. 3 shows the X-ray diffraction pattern of 5-ethoxy-2-[2-(morpholino)-ethylthio]benzimidazole dihydrochloride crystal Form II
Fig. 4 shows the FT-IR spectrum of 5-ethoxy-2-[2-(morpholino)-ethylthio]benzimidazole dihydrogenchloride crystal Form II
Fig. 5 shows the X-ray diffraction pattern of 5-ethoxy-2-[2-(morpholino)-ethylthio]benzimidazole dihydrogenchloride crystal Form III
Fig. 6 shows the FT-IR spectrum of 5-ethoxy-2-[2-(morpholino)-ethylthio]benzimidazole dihydrogenchloride crystal Form III
Fig. 7 shows the X-ray diffraction pattern of 5-ethoxy-2-[2-(morpholino)-ethylthio]benzimidazole dihydrochloride crystal Form IV
Fig. 8 shows the FT-IR spectrum of 5-ethoxy-2-[2-(morpholino)-ethylthio]benzimidazole dihydrogenchloride crystal Form IV

The present invention will be described in detail through the following examples. The examples are presented for illustrating purposes only and should not be construed as limiting the invention.

The novel polymorphs of the invention have been characterized by powder X-ray diffraction patterns using Bruker D8 Advance diffractometer (the measurement conditions: radiation, CuKα, 1.54180 Å; power, 40 kV x 40 mA; divergence slit, 1.0 mm; scattering slit, 1.0 mm; receiving slit, 0.6 mm; scan step, 0.02° 2Θ; counting time, 0.5 s/step; scan range 3-30°). The interplanar spacing's (in Angstroms) and typical relative intensities (I/I₁) sufficient to identify Forms I, II, III and IV Afobazole according to the invention set forth in Table 1 and Table 2. The novel polymorphs were further characterized by infrared (IR) spectroscopy obtained in a KBr disk using an ATI FTIR FM, ATI MATTISON as set forth in Figures 2, 4, 6 and 8. The IR absorbencies (in wavenumbers, cm⁻¹) sufficient to identify Forms I, II, III and IV.

Example 1

Preparation of 5-ethoxy-2-[2-(morpholino)-ethylthio]benzimidazole dihydrochloride polymorphic Form I

5-Ethoxy-2-[2-(morpholino)-ethylthio]benzimidazole dihydrochloride (0,5 g) was recrystallised from absolute ethanol (6,5 mL). The wet cake was dried for 6-8 hours at 60°C and Form I of 5-ethoxy-2-[2-(morpholino)-ethylthio]benzimidazole dihydrochloride was formed white crystalline powder, having water content (0.68-1.60%). The yield was 0.37 g (75%). Melting temperature 201°C.

Example 2

Preparation of 5-ethoxy-2-[2-(morpholino)-ethylthio]benzimidazole dihydrochloride polymorphic Form II

5-Ethoxy-2-[2-(morpholino)-ethylthio]benzimidazole dihydrochloride crystal Form I was heated for 8-10 hours at 80-90°C and 5-ethoxy-2-[2-(morpholino)-ethylthio]benzimidazole dihydrochloride polymorphic Form II was formed white crystalline powder, having water content (0.24%). Melting temperature 200°C.

**Table 1**

| The summary of X-Ray diffraction peaks for 5-ethoxy-2-[2-(morpholino)-ethylthio]benzimidazole dihydrochloride polymorphic Form I and Form II | | | | | |
|---|---|---|---|---|---|
| Form I | | | Form II | | |
| Angle, 2Θ | d spacings, Å | Intensity, % | Angle, 2Θ | d spacings, Å | Intensity, % |
| 5,936 | 14,87699 | 100 | 6,146 | 14,36964 | 100 |
| 7,992 | 11,05356 | 12,7 | 8,154 | 10,83453 | 26,1 |
| 10,719 | 8,24672 | 2,7 | 12,282 | 7,20071 | 32,6 |
| 11,914 | 7,42225 | 26,6 | 13,408 | 6,59845 | 7,1 |
| 13,069 | 6,76868 | 3,2 | 15,479 | 5,71977 | 10,5 |
| 15,471 | 5,72277 | 5,2 | 16,322 | 5,42638 | 11,7 |
| 16,077 | 5,50866 | 3,2 | 17,259 | 5,13389 | 9,2 |
| 16,611 | 5,33271 | 6,6 | 19,205 | 4,61784 | 20,8 |
| 17,191 | 5,15385 | 4,5 | 21,518 | 4,12643 | 9,9 |
| 18,723 | 4,73544 | 9,7 | 22,368 | 3,97142 | 8,4 |
| 19,602 | 4,52509 | 8,3 | 23,18 | 3,83412 | 4,2 |
| 21,556 | 4,11924 | 1,9 | 24,261 | 3,6656 | 10,7 |
| 21,858 | 4,06301 | 2,1 | 24,582 | 3,61847 | 8,9 |
| | | | 25,035 | 3,554 | 5,2 |
| | | | 25,663 | 3,46844 | 26,2 |
| | | | 27,665 | 3,22187 | 5,9 |
| | | | 28,45 | 3,13475 | 3,4 |
| | | | 29,238 | 3,05197 | 6,6 |

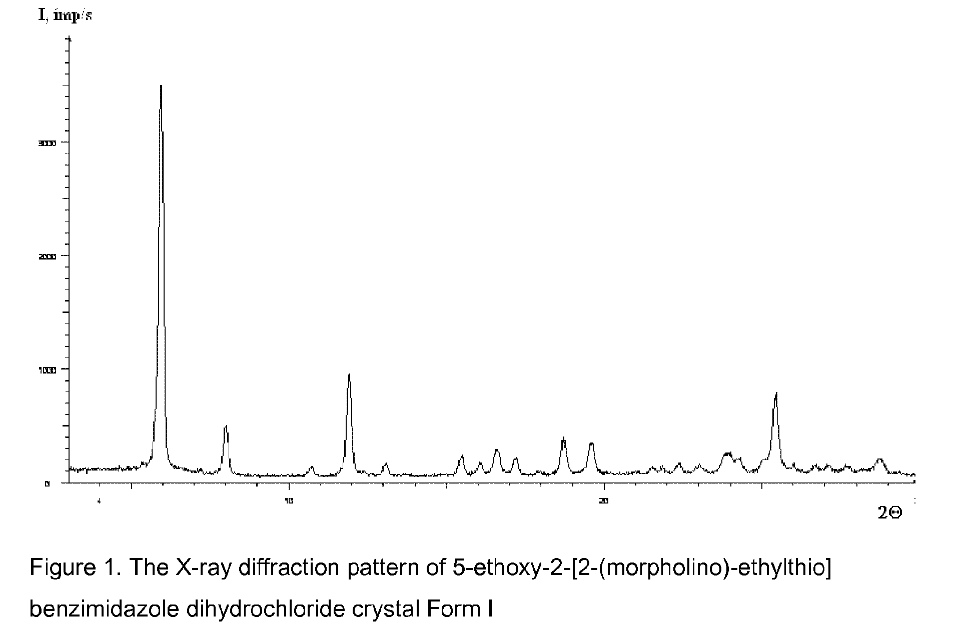

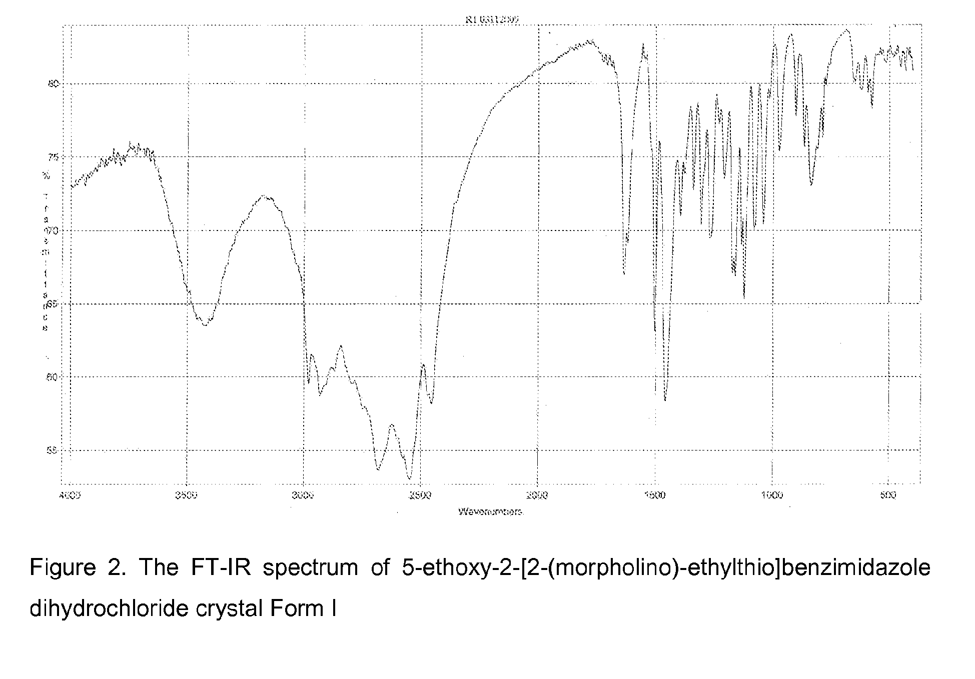

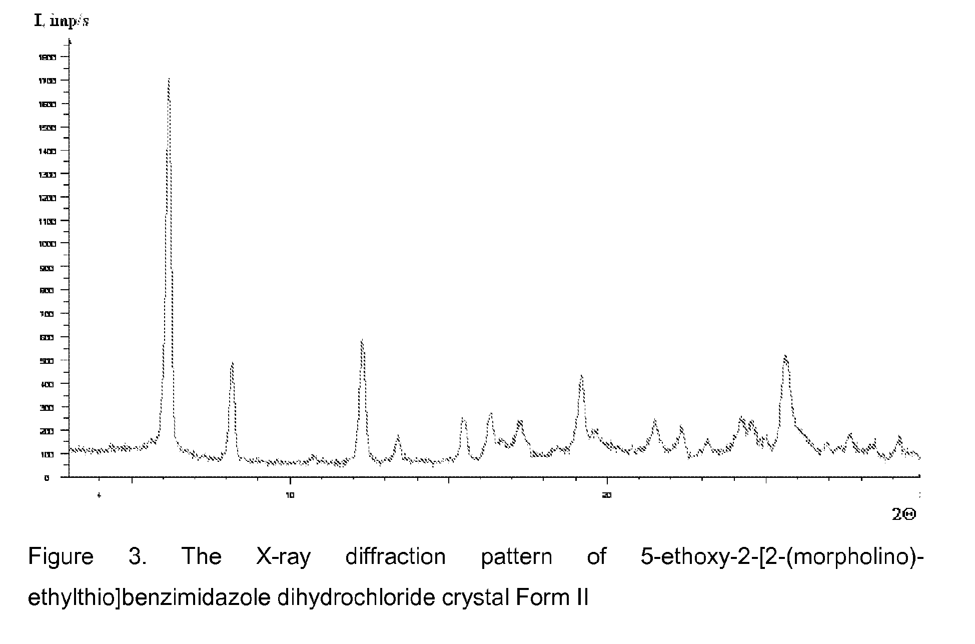

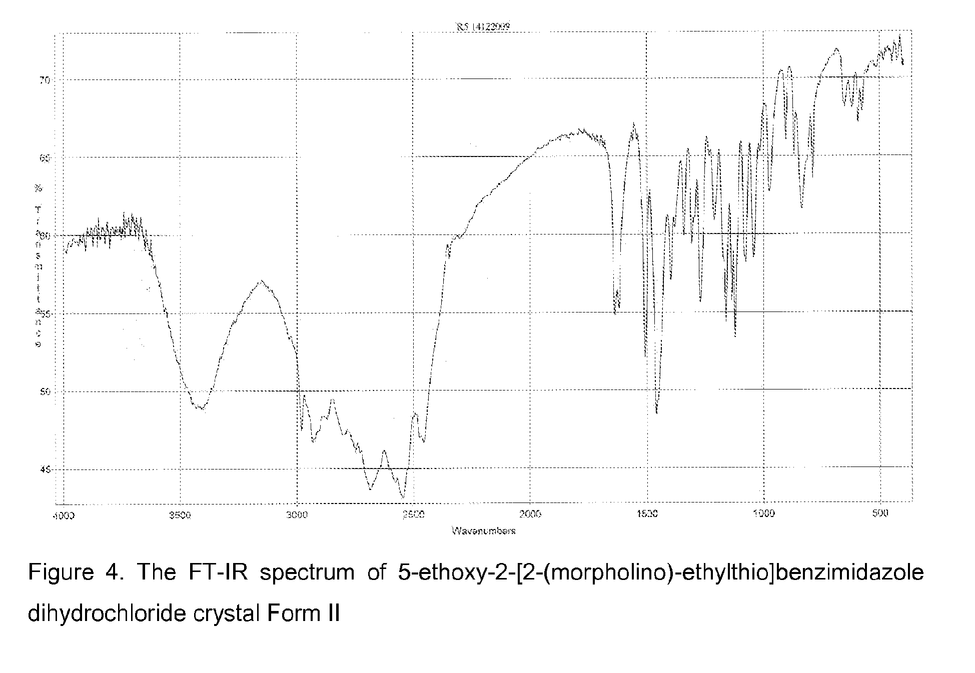

Example 3

Preparation of 5-ethoxy-2-[2-(morpholino)-ethylthio]benzimidazole dihydrogenchloride polymorphic Form III

5-Ethoxy-2-[2-(morpholino)-ethylthio]benzimidazole dihydrogenchloride Form II was treated under temperature of 25°C and relative humidity 68-70% during 24 hours, then subsequently was heated for 8-10 hours at 100°C and 5-ethoxy-2-[2-(morpholino)-ethylthio]benzimidazole dihydrogenchloride polymorphic Form III was formed as a white crystalline powder, having water content (0.97%). Melting temperature 196°C.

Example 4

Preparation of 5-ethoxy-2-[2-(morpholino)-ethylthio]benzimidazole dihydrogenchloride polymorphic Form IV

5-Ethoxy-2-[2-(morpholino)-ethylthio]benzimidazole dihydrogenchloride form (0,5 g) was added to n-butanol (1.5 mL) and heated till 118°C. The mixture was heated at 118°C for 0.5-1 hour. The wet cake was dried for 3 hours at 60°C and 5-ethoxy-2-[2-(morpholino)-ethylthio]benzimidazole dihydrogenchloride polymorphic Form IV was formed as a white crystalline powder, having water content (0.16%). The yield was 0.45 g (90%). Melting temperature 209°C.

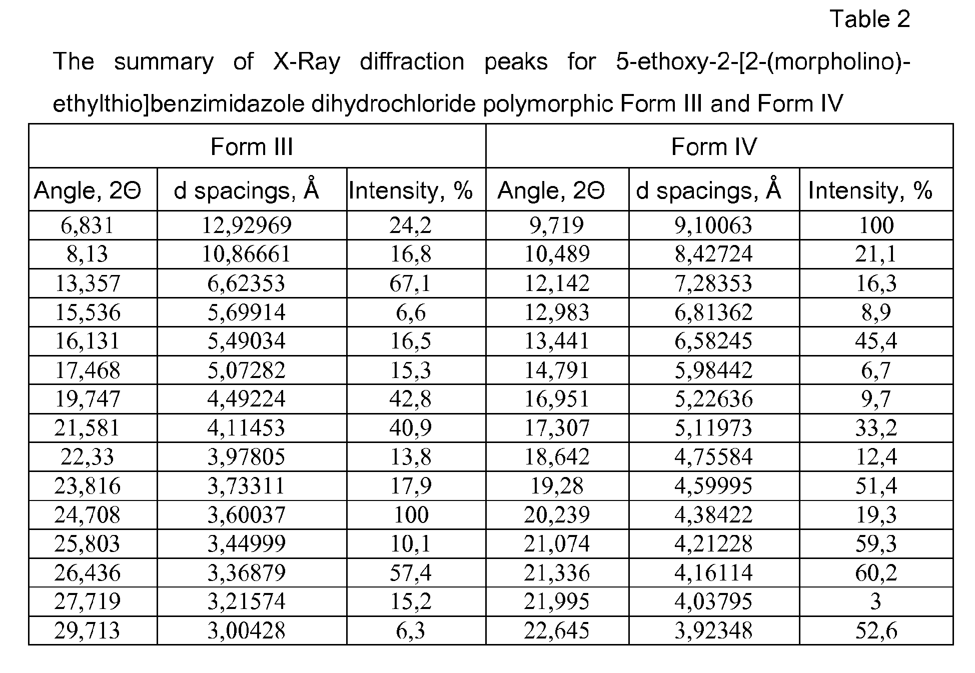

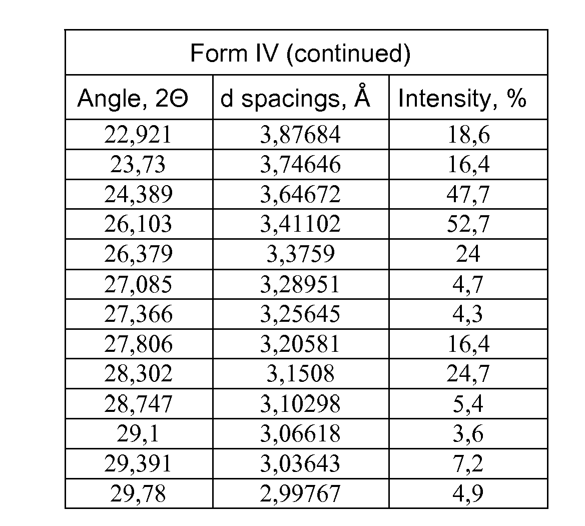

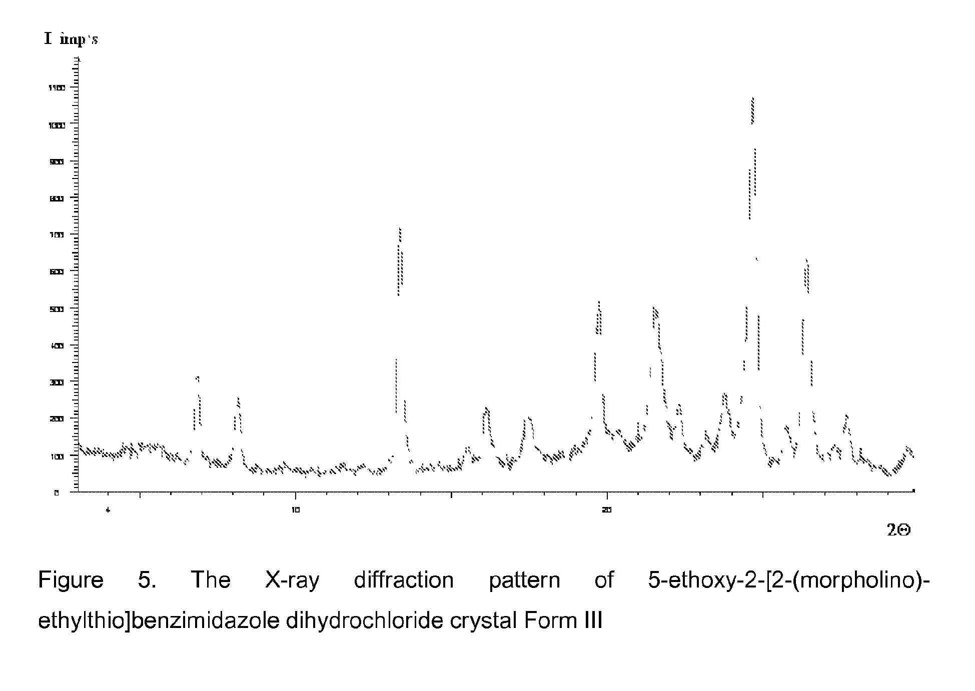

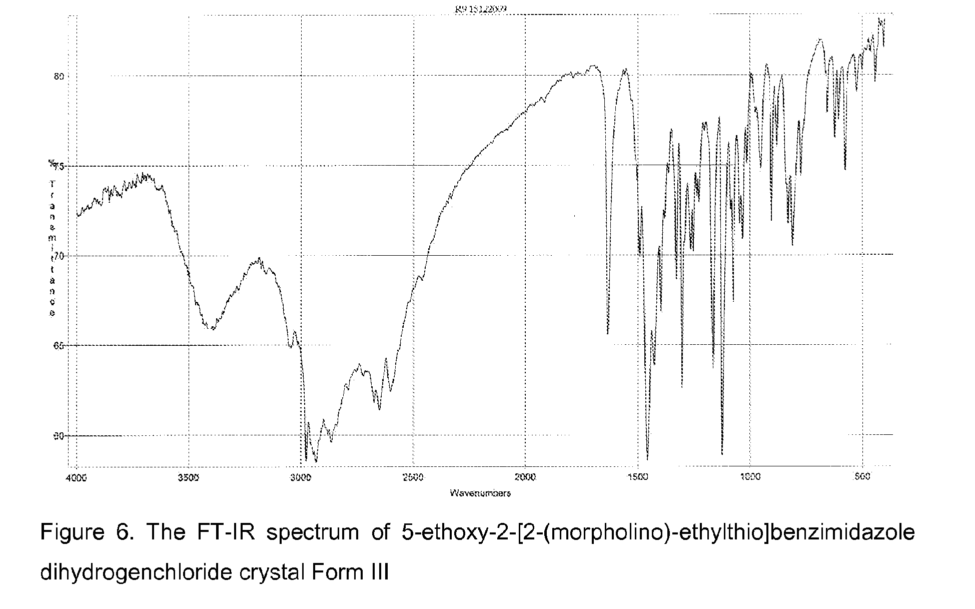

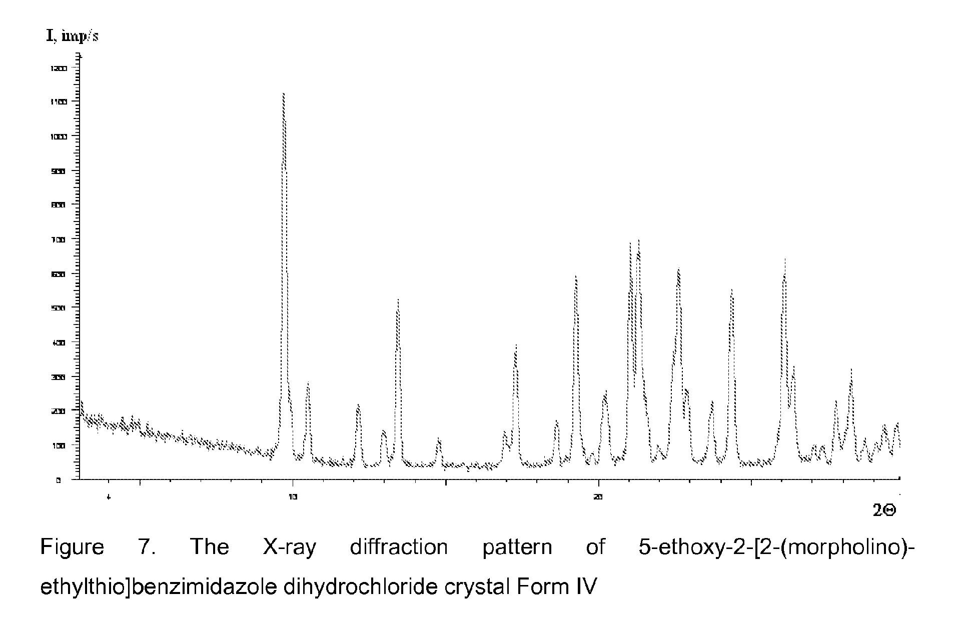

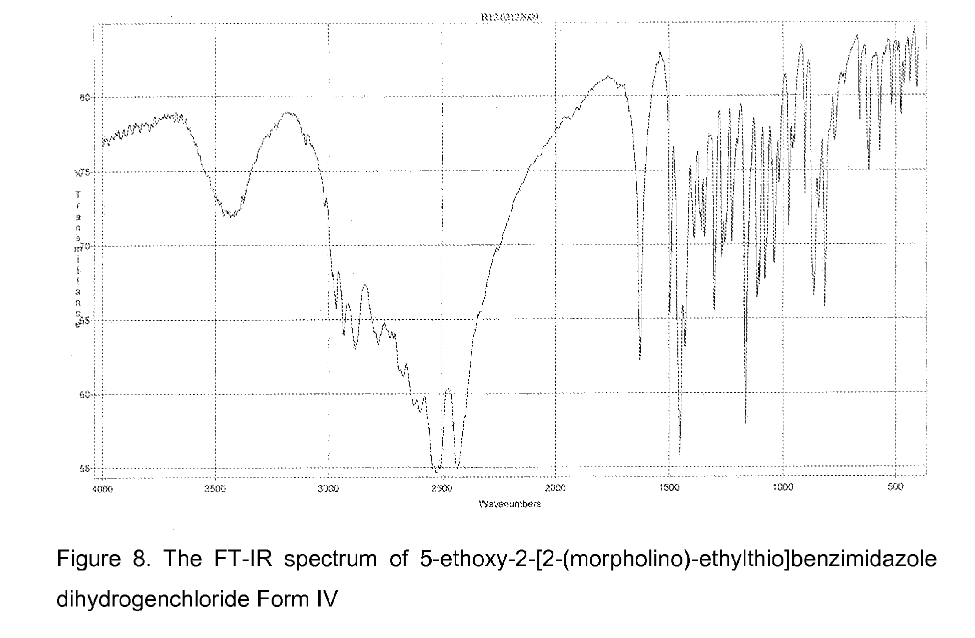

## Claims

1. 5-Ethoxy-2-[2-(morpholino)-ethylthio]benzimidazole dihydrochloride polymorphic Form I having a typical X-ray powder diffraction pattern as represented by the following:
| Angle, 2Θ | d spacings, Å | Intensity, % |
|---|---|---|
| 5,936 | 14,87699 | 100 |
| 7,992 | 11,05356 | 12,7 |
| 10,719 | 8,24672 | 2,7 |
| 11,914 | 7,42225 | 26,6 |
| 13,069 | 6,76868 | 3,2 |
| 15,471 | 5,72277 | 5,2 |
| 16,077 | 5,50866 | 3,2 |
| 16,611 | 5,33271 | 6,6 |
| 17,191 | 5,15385 | 4,5 |
| 18,723 | 4,73544 | 9,7 |
| 19,602 | 4,52509 | 8,3 |
| 21,556 | 4,11924 | 1,9 |
| 21,858 | 4,06301 | 2,1 |

2. A process for the preparation of 5-ethoxy-2-[2-(morpholino)-ethylthio]benzimidazole dihydrochloride polymorphic Form I, wherein 5-ethoxy-2-[2-(morpholino)-ethylthio]benzimidazole dihydrochloride is recrystallized from ethanol.

3. A process according to claim 2, wherein [2-(morpholino)-ethylthio]benzimidazole dihydrochloride after recrystallization is dried at 60°C.

4. A process according to claim 2 or 3, wherein [2-(morpholino)-ethylthio]benzimidazole dihydrochloride after recrystallization is dried for 6-8 hours.

5. 5-Ethoxy-2-[2-(morpholino)-ethylthio]benzimidazole dihydrochloride polymorphic Form II having a typical X-ray powder diffraction pattern as represented by the following:
| Angle, 2Θ | d spacings, Å | Intensity, % |
|---|---|---|
| 6,146 | 14,36964 | 100 |
| 8,154 | 10,83453 | 26,1 |
| 12,282 | 7,20071 | 32,6 |
| 13,408 | 6,59845 | 7,1 |
| 15,479 | 5,71977 | 10,5 |
| 16,322 | 5,42638 | 11,7 |
| 17,259 | 5,13389 | 9,2 |
| 19,205 | 4,61784 | 20,8 |
| 21,518 | 4,12643 | 9,9 |
| 22,368 | 3,97142 | 8,4 |
| 23,18 | 3,83412 | 4,2 |
| 24,261 | 3,6656 | 10,7 |
| 24,582 | 3,61847 | 8,9 |
| 25,035 | 3,554 | 5,2 |
| 25,663 | 3,46844 | 26,2 |
| 27,665 | 3,22187 | 5,9 |
| 28,45 | 3,13475 | 3,4 |
| 29,238 | 3,05197 | 6,6 |

6. A process for the preparation of 5-ethoxy-2-[2-(morpholino)-ethylthio]benzimidazole dihydrochloride polymorphic Form II, wherein 5-ethoxy-2-[2-(morpholino)-ethylthio]benzimidazole dihydrochloride polymorphic Form I is heated at 80 to 90°C.

7. A process according to claim 6, wherein 5-ethoxy-2-[2-(morpholino)-ethylthio]benzimidazole dihydrochloride polymorphic Form I is heated for 8 to 10 hours.

8. 5-Ethoxy-2-[2-(morpholino)-ethylthio]benzimidazole dihydrochloride polymorphic Form III having a typical X-ray powder diffraction pattern as represented by the following:
| Angle, 2Θ | d spacings, Å | Intensity, % |
|---|---|---|
| 6,831 | 12,92969 | 24,2 |
| 8,13 | 10,86661 | 16,8 |
| 13,357 | 6,62353 | 67,1 |
| 15,536 | 5,69914 | 6,6 |
| 16,131 | 5,49034 | 16,5 |
| 17,468 | 5,07282 | 15,3 |
| 19,747 | 4,49224 | 42,8 |
| 21,581 | 4,11453 | 40,9 |
| 22,33 | 3,97805 | 13,8 |
| 23,816 | 3,73311 | 17,9 |
| 24,708 | 3,60037 | 100 |
| 25,803 | 3,44999 | 10,1 |
| 26,436 | 3,36879 | 57,4 |
| 27,719 | 3,21574 | 15,2 |
| 29,713 | 3,00428 | 6,3 |

9. A process for the preparation of 5-ethoxy-2-[2-(morpholino)-ethylthio]benzimidazole dihydrogenchloride polymorphic Form III, wherein 5-ethoxy-2-[2-(morpholino)-ethylthio]benzimidazole dihydrogenchloride Form II is treated under relative humidity 68-70% at 25°C for 20 to 28 hours and subsequently is heated at 100°C.

10. A process according to claim 9, wherein 5-ethoxy-2-[2-(morpholino)-ethylthio]benzimidazole is heated at 100°C for 8 to 10 hours.

11. 5-Ethoxy-2-[2-(morpholino)-ethylthio]benzimidazole dihydrochloride polymorphic Form IV having a typical X-ray powder diffraction pattern as represented by the following:
| Angle, 2Θ | d spacings, Å | Intensity, % |
|---|---|---|
| 9,719 | 9,10063 | 100 |
| 10,489 | 8,42724 | 21,1 |
| 12,142 | 7,28353 | 16,3 |
| 12,983 | 6,81362 | 8,9 |
| 13,441 | 6,58245 | 45,4 |
| 14,791 | 5,98442 | 6,7 |
| 16,951 | 5,22636 | 9,7 |
| 17,307 | 5,11973 | 33,2 |
| 18,642 | 4,75584 | 12,4 |
| 19,28 | 4,59995 | 51,4 |
| 20,239 | 4,38422 | 19,3 |
| 21,074 | 4,21228 | 59,3 |
| 21,336 | 4,16114 | 60,2 |
| 21,995 | 4,03795 | 3 |
| 22,645 | 3,92348 | 52,6 |
| 22,921 | 3,87684 | 18,6 |
| 23,73 | 3,74646 | 16,4 |
| 24,389 | 3,64672 | 47,7 |
| 26,103 | 3,41102 | 52,7 |
| 26,379 | 3,3759 | 24 |
| 27,085 | 3,28951 | 4,7 |
| 27,366 | 3,25645 | 4,3 |
| 27,806 | 3,20581 | 16,4 |
| 28,302 | 3,1508 | 24,7 |
| 28,747 | 3,10298 | 5,4 |
| 29,1 | 3,06618 | 3,6 |
| 29,391 | 3,03643 | 7,2 |
| 29,78 | 2,99767 | 4,9 |

12. A process for the preparation of 5-ethoxy-2-[2-(morpholino)-ethylthio]benzimidazole dihydrochloride polymorphic Form IV, wherein 5-ethoxy-2-[2-(morpholino)-ethylthio]benzimidazole dihydrochloride is added to n-butanol.

13. A process according to claim 12, wherein [2-(morpholino)-ethylthio]benzimidazole dihydrochloride with n-butanol is heated at 115-120°C.

14. A process according to claim 12 or 13, wherein [2-(morpholino)-ethylthio]benzimidazole dihydrochloride with n-butanol is heated for 0.5-1 hour.

15. A process according to claims 12 - 14, wherein [2-(morpholino)-ethylthio]benzimidazole dihydrochloride after crystallization is dried at 60°C for 3 hours.
